# EUROPEAN PATENT APPLICATION

(11) **EP 4 071 248 A1**
(43) Date of publication of application: **12.10.2022**
(21) Application number: 21167209.2
(22) Date of filing: 07.04.2021
(51) Int. Cl.: C12N 15/87, A61K 31/506, C12N 15/867

(54) **MEANS AND METHODS FOR ENHANCING RECEPTOR-TARGETED GENE TRANSFER**

(71) Applicant: Deutsches Krebsforschungszentrum Stiftung des öffentlichen Rechts, 69120 Heidelberg (DE)
(72) Inventor: Braun, Angela, 69120 Heidelberg (DE); Frank, Annika, 60527 Frankfurt (DE); Buchholz, Christian, 60594 Frankfurt (DE)
(74) Representative: Altmann Stößel Dick Patentanwälte PartG mbB

(57) **Abstract**

The present invention concerns the field of gene transfer. More specifically, the present invention relates to method for enhancing receptor-targeted gene transfer into a primary T-cell as a host cell said method comprising contacting a host cell comprised in a sample with a gene transfer vehicle comprising a targeting agent which specifically binds to CD3, CD4 or CD8 and a nucleic acid of interest to be transferred into the host cell in the presence of a tyrosine kinase inhibitor which is capable of inhibiting the LCK tyrosine kinase in said host cell and cultivating said host cell culture obtained for a time and under conditions which allow for receptor-targeted gene transfer. The present invention also relates to a method for the preparation of a medicament as well as a medicament comprising a gene transfer vehicle comprising a targeting agent which specifically binds to CD3, CD4 or CD8 and a nucleic acid of interest to be transferred into primary T-cells and a tyrosine kinase inhibitor which is capable of inhibiting the LCK tyrosine kinase in said primary T-cells. Furthermore, also relates to a tyrosine kinase inhibitor for use in receptor-targeted gene transfer into said primary T-cell in a subject being in need thereof, wherein said tyrosine kinase inhibitor is capable of inhibiting the LCK tyrosine kinase in a primary T-cell as a host cell or gene transfer vehicle for use in enhancing receptor-targeted gene transfer into said primary T-cell in a subject being in need thereof, wherein the gene transfer vehicle is used in combination with a tyrosine kinase inhibitor which is capable of inhibiting the LCK tyrosine kinase in a primary T-cell as a host cell.

## Description

The present invention concerns the field of gene transfer. More specifically, the present invention relates to method for enhancing receptor-targeted gene transfer into a primary T-cell as a host cell said method comprising contacting a host cell comprised in a sample with a gene transfer vehicle comprising a targeting agent which specifically binds to CD3, CD4 or CD8 and a nucleic acid of interest to be transferred into the host cell in the presence of a tyrosine kinase inhibitor which is capable of inhibiting the LCK tyrosine kinase in said host cell and cultivating said host cell culture obtained for a time and under conditions which allow for receptor-targeted gene transfer. The present invention also relates to a method for the preparation of a medicament as well as a medicament comprising a gene transfer vehicle comprising a targeting agent which specifically binds to CD3, CD4 or CD8 and a nucleic acid of interest to be transferred into primary T-cells and a tyrosine kinase inhibitor which is capable of inhibiting the LCK tyrosine kinase in said primary T-cells. Furthermore, also relates to a tyrosine kinase inhibitor for use in receptor-targeted gene transfer into said primary T-cell in a subject being in need thereof, wherein said tyrosine kinase inhibitor is capable of inhibiting the LCK tyrosine kinase in a primary T-cell as a host cell or gene transfer vehicle for use in enhancing receptor-targeted gene transfer into said primary T-cell in a subject being in need thereof, wherein the gene transfer vehicle is used in combination with a tyrosine kinase inhibitor which is capable of inhibiting the LCK tyrosine kinase in a primary T-cell as a host cell.

Various life-threatening diseases such as cancer, immunological diseases and metabolic or neurological disease require treatment by gene therapy or immunotherapy.

Different gene transfer vehicles have been reported for gene transfer in gene therapeutic approaches. Among those vehicles there are retroviral vectors and, in particular, lentiviral vectors, as well as adenoviral or adenovirus-like vectors or non-viral vehicles such as liposomes or nanoparticles.

Lentiviral vectors (LVs) are important tools in gene therapy and immunotherapy, since they have the potential to increase safety and to simplify the complex manufacturing process of T-cell therapy products such as T-cells expressing chimeric antigen receptors (CARs) (Jamali et al 2019). Moreover, they have made direct in vivo generation of CAR T-cells possible (Pfeiffer et al. 2018; Aarwal et al. 2020; Frank et al. 2020). However, the T-cell-targeted LVs are characterized by lower transduction efficiencies compared to conventionally used vectors.

So far, transduction of T-cells in vitro by T-cell-targeted LVs has been increased by transduction enhancers such as Vectofusin-1 (Jamali, Kapitza et al. , 2019).

Vectofusin-1 yields a significant increase in transduction by T-cell-targeted LVs in vitro but it has not yet been tested in in vivo models. It remains to be determined whether the enhancer can be safely used for such applications, and whether it retains its transduction enhancing properties also upon in vivo use.

Dasatinib is an FDA and EMA approved drug used as a therapy or AML and CML, its biodistribution and kinetics in vivo as well as potential side effects are well known. Furthermore, dasatinib has been shown to control CAR T-cell activation in vitro and in vivo (Mestermann, 2019).

Dasatinib is a member of the Src/Abl tyrosine kinase inhibitor family known to interfere with the T-cell specific protein tyrosine kinase LCK, an upstream kinase in the T-cell activation cascade. By blocking LCK phosphorylation of the CD3-zeta domain and continuously ZAP 70 are inhibited, preventing activation-induced downregulation of the T-cell receptor (TCR) (Schade et al. 2008; Lissina et al. 2009). Thereby, TCR surface levels are increased in presence of the inhibitor. Similar effects of dasatinib treatment have also been observed with the CD8 co-receptor, although the exact mechanism behind this phenomenon remains to be further studied (Lissina et al. 2009).

The use of tyrosine kinase inhibitors and, in particular those of the Src/Abl tyrosine kinase inhibitor family, before, during and after targeted gene therapy has been described (see, e.g., Shangming et al., 2006, WO2019/110815, WO2020/092650, Wo2019/213184, WO2018/183842, US2020/181573). However, there are no reports on transducing enhancing properties of such tyrosine kinase inhibitors.

As described above, means for increasing the transducing efficacy for gene transfer would be highly desired for both, in vitro and in vivo applications.

The technical problem underlying the present invention may be seen as the provision of means and methods for complying with the aforementioned needs. The technical problem is solved by the embodiments characterized in the claims and herein below.

Thus, the present invention relates to a method for enhancing receptor-targeted gene transfer into a primary T-cell as a host cell said method comprising:
(a) contacting a host cell comprised in a sample with a gene transfer vehicle comprising a targeting agent which specifically binds to CD3, CD4 or CD8 and a nucleic acid of interest to be transferred into the host cell in the presence of a tyrosine kinase inhibitor which is capable of inhibiting the LCK tyrosine kinase in said host cell; and
(b) cultivating said host cell culture obtained in step (a) for a time and under conditions which allow for receptor-targeted gene transfer.

It is to be understood that in the specification and in the claims, "a" or "an" can mean one or more, depending upon the context in which it is used. Thus, for example, reference to "an" item can mean that at least one item can be utilized.

As used in the following, the terms "have", "comprise" or "include" are meant to have a non-limiting meaning or a limiting meaning. Thus, having a limiting meaning these terms may refer to a situation in which, besides the feature introduced by these terms, no other features are present in an embodiment described, i.e. the terms have a limiting meaning in the sense of "consisting of' or "essentially consisting of'. Having a non-limiting meaning, the terms refer to a situation where besides the feature introduced by these terms, one or more other features are present in an embodiment described.

Further, as used in the following, the terms "preferably", "more preferably", "most preferably", "particularly", "more particularly", "typically", and "more typically" or similar terms are used in conjunction with additional or alternative features, without restricting alternative possibilities.

Further, it will be understood that the term "at least one" as used herein means that one or more of the items referred to following the term may be used in accordance with the invention. For example, if the term indicates that at least one item shall be used this may be understood as one item or more than one item, i.e. two, three, four, five or any other number. Depending on the item the term refers to the skilled person understands as to what upper limit the term may refer, if any.

The method according to the present invention may consist of the aforementioned steps (a) and (b) or may comprise further steps, such as steps pre-culturing and/or pretreating the host cell prior to step (a) as well as steps for treating or further cultivating the host cell after receptor-targeted gene transfer such as selection steps and/or steps of analyzing the said receptor-targeted gene transfer.

The term "receptor-targeted gene transfer" as used herein refers to a process for introducing a nucleic acid molecule of interest (i.e. a "gene") into a host cell by using a gene transfer vehicle for gene transfer. The process is receptor-targeted, i.e. it depends, inter alia, on the specific binding between the targeting agent on the gene transfer vehicle and the CD3, CD4 or CD8 on the host cell. Due said targeting, only the targeted host cells will be affected by the gene transfer.

The term "gene transfer vehicle" as used herein refers to any vehicle which allows for gene transfer into a host cell in accordance with the present invention. Preferably, a gene transfer vehicle in accordance with the present invention is a viral gene transfer vehicle, more preferably, a retroviral-, typically lentiviral-, adenoviral-associated- or adenoviral- vectors. Also preferably, the gene transfer vehicle may be a virus-like particle, liposome or liposomebased vehicle or a nanoparticle. The gene transfer vehicle according to the present invention shall exhibit on its surface a targeting agent which specifically binds to CD3, CD4 or CD8, preferably, to CD3. This targeting agent shall be capable of specifically binding to the targeting structure on the host cell, i.e. CD3, CD4 or CD8, such that the gene targeting vehicle can internalized by the host cell and gene transfer of the a nucleic acid of interest to be transferred into the host cell may occur.

More preferably, the gene transfer vehicle according to the present invention is a lentiviral vector system. Lentiviral vectors useful for mediators of gene transfer are well known in the art. Typically, lentiviral vectors used for gene transfer in therapeutic applications are based on HIV-1. HIV-2, simian immunodeficiency viruses or non-primate lentiviruses, preferably, feline immunodeficiency virus, equine infectious anemia virus, bovine immunodeficiency virus or caprine arthritis-encephalitis virus have been also used for generating lentiviral vectors.

Such lentiviral vectors are, typically, important tools in gene therapy and immunotherapy, since they have the potential to increase safety and to simplify the complex manufacturing process of T-cell therapy products such as T-cells expressing chimeric antigen receptors (CARs) (Jamali et al 2019). Moreover, they have made direct in vivo generation of CAR T-cells possible (Pfeiffer et al. 2018; Agarwal et al. 2020; Frank et al. 2020). However, the T-cell-targeted lentiviral vectors are characterized by lower transduction efficiencies compared to conventionally used vectors.

The lentiviral vector used for gene transfer in accordance with the method of the present invention typically encompasses a vector systems comprising plasmids carrying the necessary viral components as well as a plasmid comprising the nucleic acid of interest to be transferred.

Particular preferred lentiviral vectors to be used in accordance with the present invention are described in more detail in the accompanying Examples below.

The term "targeting agent which specifically binds to CD3, CD4 or CD8" as used herein refers to a molecule which is capable of specifically recognizing and binding to one of the cell surface antigens CD3, CD4 or CD8, preferably, the extracellular part thereof.

Preferably, the targeting agent is an antibody or antibody fragment specifically binding to CD3, CD4 or CD8. Antibodies as meant in accordance with the present invention encompass to all types of antibodies which specifically bind CD3, CD4 or CD8. Preferably, an antibody of the present invention may be a monoclonal antibody, a polyclonal antibody, a single chain antibody, a chimeric antibody or any fragment of such antibodies being still capable of specifically binding to CD3, CD4 or CD8. Such fragments comprised by the term antibody as used herein encompass a bispecific antibody, a synthetic antibody, an Fab, F(ab)₂ Fv or scFv fragment or a chemically modified derivative of any of these antibody fragments. Antibodies or fragments thereof, in general, which specifically bind to a desired target can be obtained by using methods which are described, e.g., in Harlow and Lane "Antibodies, A Laboratory Manual", CSH Press, Cold Spring Harbor, 1988. Monoclonal antibodies can be prepared by the techniques which comprise the fusion of mouse myeloma cells to spleen cells derived from immunized mammals and, preferably, immunized mice. The skilled person is well aware of how specific binding can be tested by techniques well known in the art, such as immunological or biochemical techniques including immunoassays, cell sorting or Western blotting or plasmon surface resonance measurements.

Also preferably, the targeting agent in accordance with the present invention may be an aptamer, more preferably, an oligonucleic acid or peptide aptamer molecule that specifically binds to CD3, CD4 or CD8. Oligonucleic acid aptamers are engineered through repeated rounds of selection or the so-called systematic evolution of ligands by exponential enrichment (SELEX technology). Peptide aptamers usually comprise of a variable peptide loop attached at both ends to a protein scaffold. This double structural constraint shall increase the binding affinity of the peptide aptamer into the nanomolar range. Said variable peptide loop length is, preferably, composed of ten to twenty amino acids, and the scaffold may be any protein having improved solubility and compacity properties, such as thioredoxin-A. Peptide aptamer selection can be made using different systems including, e.g., the yeast two-hybrid system. The skilled person is well aware of how specific binding can be tested by techniques well known in the art, such as plasmon surface resonance measurements.

Moreover, also preferably, the targeting agent may be a protein or fragment thereof which naturally binds to the extracellular part of CD3, CD4 or CD8. Such proteins encompass typically MHC I or fragments thereof, MHC II or fragments thereof which are capable of specifically binding to CD3, CD4 or CD8.

Further preferably, the targeting agent may be a Designed Ankyrin Repeat Protein (DARPin). DARPins are genetically engineered antibody mimetic proteins which can be designed to achieve highly specific and high-affinity target protein binding. They are derived from natural ankyrin repeat proteins. Typically, DARPins comprise at least three repeat modules, of which the most N- and the most C-terminal modules (also referred to as "caps") protect the hydrophobic core of the protein (Binz et al. 2003).

The term "nucleic acid of interest to be transferred into the host cell" refers in accordance with the present invention to any nucleic acid, e.g., DNA or RNA, which shall be introduced into the host cell, i.e. the primary T-cell. Preferably, the nucleic acid of interest is a therapeutically effective nucleic acid of interest which shall be expressed in the host cell in order achieve a therapeutic effect. Accordingly, biological functions of the primary T-cell which is affected by receptor- targeted gene transfer may shall be altered, e.g. activated or silenced as a result of the expression of the nucleic acid of interest to be transferred.

The term "enhanced" as used herein refers to an increased transduction efficiency of receptor-targeted gene transfer compared to a reference efficiency which is observed for receptor-targeted gene transfer in a method were the host cell comprised in a sample is contacted with a gene transfer vehicle as described before comprising a nucleic acid of interest to be transferred into the host cell in the absence presence of a tyrosine kinase inhibitor which is capable of inhibiting the LCK tyrosine kinase in said host cell. The transduction efficiency of receptor-targeted gene transfer can be determined by a method known in the art and, preferably, as described elsewhere herein and, more preferably, as described in the accompanying Examples, below. Preferably, the term as used herein refers to an increase in transduction efficiency which is statistically significant. Whether an increase is statistically significant can be determined by standard techniques of statistics including, e.g., Student's t-test or Mann-Whitney test. Preferably, said receptor-targeted gene transfer into a primary T-cell as a host cell is enhanced at least 2-fold, at least 3 fold, at least 4 fold, up to 10 fold, preferably, at least 3 fold, compared to a control wherein the host cell was not contacted in the presence of said tyrosine kinase inhibitor.

The term "tyrosine kinase inhibitor which is capable of inhibiting the LCK tyrosine kinase in said host cell" refers to a tyrosine kinase inhibitor that is capable to reduce or block the tyrosine kinase activity of LCK in a host cell. A reduced activity as referred to herein is, preferably, a statistically significant reduced activity. Preferably, the tyrosine kinase inhibitor in accordance with the present invention is a small molecule, more preferably, a 2-aminothiazole-5- aromatic carboxamide or derivative thereof. Techniques for identifying tyrosine kinase inhibitors being inhibitors of LCK tyrosine kinase are well known in the art. Moreover, preferably, said tyrosine kinase inhibitor is a Src/Abl tyrosine kinase inhibitor as well. Techniques for identifying tyrosine kinase inhibitors as inhibitors of Src/Abl tyrosine kinase are also well known in the art. Suitable tyrosine kinase inhibitors which can be used in accordance with the present invention are disclosed in WO2005077945 or WO2006121742. Yet, the compounds disclosed in these documents may be also used for identifying tyrosine kinase inhibitors according to the present invention by determining whether a candidate compound inhibits the LCK tyrosine kinase. The tyrosine kinase inhibitor according to the present invention may be further identified by determining whether a candidate compound inhibits Src/Abl kinase.

More preferably, the tyrosine kinase inhibitor in accordance with the present invention is 'N-(2-Chloro-6-methylphenyl)-2-[[6-[4-(2-hydroxyethyl)-1-piperazinyl]-2-methyl-4-pyrimidinyl] amino]-5-thiazolecarboxamide or a salt thereof also known as dasatinib. Also preferably, said tyrosine kinase inhibitor is 4-[(4-Methylpiperazin-1-yl)methyl]-*N*-{4-methyl-3-[(4-pyridin-3-ylpyrimidin-2-yl)amino]phenyl}benzamid or a salt thereof (imatinib) or 4-Methyl-N-[3-(4-methylimidazol-1-yl)-5-(trifluormethyl)phenyl]-3-[(4-pyridin-3-ylpyrimidin-2-yl)amino] benzamid or a salt thereof (nilotinib).

The host cell for receptor-targeted gene transfer in accordance with the present invention shall be a primary T-cell. The term "primary T-cell" as used herein refers to any T-cell which can be obtained from an organism in possession of T-cells. Typically, said organism is an animal, preferably, a mammal, more preferably a laboratory animal such as a mouse, a rat, a monkey or a rabbit or a pet or farming animal such as a dog, a cat, a horse, a cow, a sheep or a goat and, most preferably, a human. It will be understood that the said primary T-cell has not been genetically modified in order to be immortalized. Primary T-cells may be cytotoxic T-cells, T helper cells, regulatory T-cells, natural killer T-cells, T memory cells, or γδ T-cells. Typically, the primary T-cell may be selected from the group consisting of: CD8 positive primary T-cells, CD4 positive primary T-cells, CD3 positive primary T-cells. More preferably, it is a CD3-positive primary T-cell.

How such primary T-cells can be obtained from an organism is well known in the art. Typically, primary T-cells can be purified or isolated by, e.g., cell sorting techniques from blood samples obtained form said organism. The primary T-cells may be cultivated after isolation or purification in a T-cell culture medium as described in the accompanying Examples below.

In the method of the present invention, a primary T-cell as a host cell comprised in a sample such as a primary cell culture is contacted with a gene transfer vehicle as described elsewhere herein comprising a gene to be transferred into the host cell. Contacting as used herein refers to bringing the two components, i.e. the host cell and the gene transfer vehicle into physical proximity such the gene transfer vehicle can act as a vector for the envisaged gene transfer.

Typically, a gene transfer vehicle preparation is added to the host cell comprised in the sample. A preferred technique for obtaining and/or cultivating primary T-cells as host cells is described in the accompanying Examples, below.

Said contacting shall be carried out in the presence of a tyrosine kinase inhibitor which is capable of inhibiting the LCK tyrosine kinase in said host cell. The tyrosine kinase inhibitor may, preferably, be already present in the sample comprising the host cell prior to contacting said host cell with the gene transfer vehicle for the envisaged gene transfer. Thus, cultivating a primary host cell culture as referred to herein prior to adding the gene transfer vehicle to the culture may be done already in the presence of the tyrosine kinase inhibitor. Preferably, the tyrosine kinase inhibitor is added 3 h, 2 h, 1 h, 30 min, 20 min, 10 min or 5 min prior to contacting the host cell to the gene transfer vehicle.

Also preferably, the said tyrosine kinase inhibitor may be added to the sample comprising the host cell together with the gene transfer vehicle for the envisaged gene transfer or after the host cell has been contacted to the gene transfer vehicle. In the latter case, it is, however, envisaged that the gene transfer takes place in the presence of the tyrosine kinase inhibitor. Thus, if the tyrosine kinase inhibitor is added after the host cell has been contacted with the gene transfer vehicle, the addition of the tyrosine kinase inhibitor must be done shortly after said contact, e.g. after 5 min, 10 min or 20 min. A preferred contacting is described in the accompanying Examples, below.

After the step of contacting as described herein above, the host cell in the culture obtained after the contacting step is to be cultivated for a time and under conditions which allow for receptor-targeted gene transfer. Typically, the cultivation is done under essentially the same cultivation conditions as the cultivation of the host cell prior to contacting the said host cell to the gene transfer vehicle, i.e. cultivation conditions for primary T-cells as host cells. Preferably, the cultivation takes place for a first cultivation period of at least 1 h, at least 2 h, at least 3 h, at least 4 h, at least 5 h, at least 6 h, at least 7 h, at least 8 h at least 9 h or up to 10 h in the presence of the tyrosine kinase inhibitor according to the invention. After said first cultivation period in the presence of the tyrosine kinase inhibitor, the cultivation is continued in the absence of the tyrosine kinase inhibitor according to the invention. Said second cultivation period may, preferably, be at least 1 day, at least 2 days, at least 3 days, at least 4 days, at least 5 days, at least 6 days, at least 1 week or at least 2 weeks. Preferred culture conditions are described in the accompanying Examples, below.

During the first and/o the second cultivation period and/or during contacting, further agents, such as transduction enhancers, may be present which facilitate receptor-targeted gene transfer in the host cell. Accordingly, it is preferably envisaged in the method of the invention that said host cell is contacted during step (a) and/or step (b) with at least one further transduction enhancer. More preferably, said further transduction enhancer is selected from the group consisting of: Vectofusin-1, BX 795, Cyclosporin A, CyclosporinH, 16,16-Dimethyl Prostaglandin E2, Prostaglandin E2, Rapamycin, Rosuvastin calcium, and Staurosporine.

Advantageously, it has been found in the studies underlying the present invention that tyrosine kinase inhibitors such as the Src/Abl tyrosine kinase inhibitor dasatinib are potent enhancers of gene delivery into lymphocytes, typically, mediated by T-cell-targeted lentiviral vectors as gene transfer vehicles. In particular, it was demonstrated that pretreatment of T-cells with dasatinib dramatically increases cell-directed gene transfer in vitro using CD3-specific LVs (CD3LVs). Dasatinib. pretreatment resulted in substantially and unexpectedly increased gene transfer rates into T-cells of 3- to up to 10-fold in comparison to untreated control cells without affecting vector selectivity or cellular integrity. Moreover, transduction enhancement by dasatinib is active on both, T-cells stimulated with recombinant anti-CD3 and anti-CD28 antibodies, and T-cells cultured only in presence of cytokines. On fully activated PBMC, CD3-L Vs were able to transduce up to 70% of T-cells, in presence of dasatinib, which is comparable to transduction rates observed for the commonly used VSV-LV under optimal conditions. As recently described, CD3-LVs are able to transduce cytokine-only stimulated T-cells which could be improved 7-fold in presence of dasatinib. VSV-LV transduction of cytokine-only stimulated T-cells, however, does not result in significant levels of transduced cells.

Thanks to the findings underlying the present invention exclusive gene delivery with high efficiency into primary human lymphocytes is enabled. T-cell-targeting gene transfer vehicles ensure exclusive transduction of the T-cell target population while other cell types remain unaffected. It will be understood that the use of a clinically approved agent such as the tyrosine kinase inhibitor dasatinib is in such an approach also straightforward with regard to in vivo applications. The increase in transduction caused by dasatinib was even stronger than by the transduction enhancer Vectofusin-1. Dasatinib can synergize with Vectofusin since it mode of action is completely different and independent, resulting in even further increased transduction efficiency.

The present invention also relates to a method for the preparation of a cellular therapeutic composition comprising the steps of the any one of the aforementioned methods for enhancing receptor-targeted gene transfer into a primary T-cell as a host cell and the further step of formulating the host cell culture obtained in step (b) as a cellular therapeutic composition.

It will be understood that the host cell culture or isolated host cells thereof which have been obtained by the aforementioned methods for enhancing receptor-targeted gene transfer into a primary T-cell as a host cell can be further formulated as a cellular therapeutic composition for treating diseases. The cells comprised in the cellular therapeutic composition are typically present in a pharmaceutically acceptable solution or diluent which can be applied to a subject and which does not cause any damage to the cells. The cells of the cellular therapeutic composition are viable cells that are biologically active.

Preferably, said cellular therapeutic composition is for treating (i) cancer, preferably leukemia, more preferably lymphoblastic leukemia, or (ii) an infectious disease, preferably viral infection, more preferably, HIV infection.

The term "cancer" as referred to herein relates to a class of disease involving abnormal and uncontrolled cell growth. Preferably, the cancer in accordance with the present invention is a cancer which is suitable for treatment by gene- or immunotherapy such as lymphatic cancer, lung cancer, breast cancer, colon cancer, kidney cancer, prostate cancer, cancer in the CNS, skin cancer. More preferably, the cancer is a lymphatic cancer, typically, leukemia, most preferably, lymphoblastic leukemia.

The term "infectious disease" as referred to herein relates to any infection caused a pathogenic invader such as a bacteria or viruses. Other pathogenic invaders may be fungi or uni- or multicellular parasites such as worms, helminths or arthropods. Preferably, the infectious disease according to the invention is a viral infection, more preferably, a HIV infection.

The present invention also relates to a medicament comprising:
(a) a gene transfer vehicle comprising a targeting agent which specifically binds to CD3, CD4 or CD8 and a nucleic acid of interest to be transferred into primary T-cells; and
(b) tyrosine kinase inhibitor which is capable of inhibiting the LCK tyrosine kinase in said primary T-cells.

The term "medicament" as used herein refers to a pharmaceutical composition comprising the aforementioned ingredients i.e. (i) the gene transfer vehicle for enhancing receptor-targeted gene transfer into a primary T-cell as a host cell and (ii) the tyrosine kinase inhibitor which is capable of inhibiting the LCK tyrosine kinase in said primary T-cells as pharmaceutical active compounds.
Preferably, said tyrosine kinase inhibitor is a tyrosine kinase inhibitor as defined elsewhere herein. Said medicament may be used for human or non-human therapy of various diseases or disorders as specified elsewhere herein in a therapeutically effective dose. The gene transfer vehicle and the tyrosine kinase inhibitor may be present in premixed form or as separate compounds in the medicament according to the invention. Typically, both are present in a premixed form which may be applied to the patient in need of gene transfer therapy. The medicament is, preferably, for topical or systemic administration. Conventionally a medicament will be administered intra-muscular or subcutaneous. However, depending on the nature and the desired therapeutic effect and the mode of action, the medicament may be administered by other routes as well. The medicament is, preferably, administered in conventional dosage forms prepared by combining the ingredients with standard pharmaceutical carriers according to conventional procedures. These procedures may involve mixing or dissolving the ingredients as appropriate to the desired preparation. Preferably, a solution is envisaged for the medicament. It will be appreciated that the form and character of the pharmaceutical acceptable carrier is dictated by the amount of active ingredient with which it is to be combined, the route of administration and other well-known variables.

A carrier must be acceptable in the sense of being compatible with the other ingredients of the formulation and being not deleterious to the recipient thereof. The pharmaceutical carrier employed may include a solid, a gel, or a liquid. Examples for solid carriers are lactose, terra alba, sucrose, talc, gelatin, agar, pectin, acacia, magnesium stearate, stearic acid and the like. Exemplary of liquid carriers are phosphate buffered saline solution, syrup, oil, water, emulsions, various types of wetting agents, are distilled water, physiological saline, Ringer's solutions, dextrose solution, and Hank's solution, and the like. Similarly, the carrier may include time delay material well known to the art, such as glyceryl mono-stearate or glyceryl distearate alone or with a wax. Said suitable carriers comprise those mentioned above and others well known in the art, see, e.g., Remington's Pharmaceutical Sciences, Mack Publishing Company, Easton, Pennsylvania.

In addition, the medicament may also include other carriers, adjuvants, or non-toxic, non-therapeutic, non-immunogenic stabilizers and the like.

A therapeutically effective dosage of the tyrosine kinase inhibitor refers to an amount of the tyrosine kinase inhibitor to be used in medicament of the present invention which enhances receptor-targeted gene transfer. A therapeutically effective dosage of the gene transfer vehicle for receptor-targeted gene transfer refers to an amount of gene transfer vehicle that prevents, ameliorates or treats the symptoms accompanying a disease or condition referred to in this specification. Therapeutic efficacy and toxicity of the compound can be determined by standard pharmaceutical procedures in cell cultures or experimental animals, e.g., ED50 (the dose therapeutically effective in 50% of the population) and LD50 (the dose lethal to 50% of the population). The dose ratio between therapeutic and toxic effects is the therapeutic index, and it can be expressed as the ratio, LD50/ED50. The dosage regimen will be determined by the attending physician and other clinical factors. As is well known in the medical arts, dosages for any one patient depends upon many factors, including the patient's size, body surface area, age, the particular compound to be administered, sex, time and route of administration, general health, and other drugs being administered concurrently. Progress can be monitored by periodic assessment. The medicament referred to herein is administered at least once in order to treat or ameliorate or prevent a disease or condition recited in this specification. However, the said medicament may be administered more than one time.

The medicament according to the present invention may also comprise drugs or other ingredients which are added to the medicament during its formulation. Preferably, said medicament further comprises an transduction enhancer as defined elsewhere herein.

Finally, it is to be understood that the formulation of a medicament takes place under GMP standardized conditions or the like in order to ensure quality, pharmaceutical security, and effectiveness of the medicament.

The present invention also relates to a tyrosine kinase inhibitor for use in enhancing receptor-targeted gene transfer into a primary T-cell in a subject being in need thereof, wherein said tyrosine kinase inhibitor is capable of inhibiting the LCK tyrosine kinase in a primary T-cell as a host cell and wherein a gene transfer vehicle comprising a targeting agent which specifically binds to CD3, CD4 or CD8 and a nucleic acid of interest to be transferred into the host cell.

Preferably, said tyrosine kinase inhibitor to be used is a tyrosine kinase inhibitor as defined elsewhere herein. Preferably, said tyrosine kinase inhibitor is to be used in combination with the transduction enhancer as defined elsewhere herein.

Preferably, said subject being in need of receptor-targeted gene transfer suffers from (i) cancer, preferably leukemia, more preferably lymphoblastic leukemia, or (ii) an infectious disease, preferably viral infection, more preferably, HIV infection.

Also provided in accordance with the preset invention is a gene transfer vehicle comprising a targeting agent which specifically binds to CD3, CD4 or CD8 and a nucleic acid of interest to be transferred into the host cell for use in enhancing receptor-targeted gene transfer into a primary T-cell in a subject being in need thereof, wherein the gene transfer vehicle is used in combination with a tyrosine kinase inhibitor which is capable of inhibiting the LCK tyrosine kinase in a primary T-cell as a host cell.

Preferably, said tyrosine kinase inhibitor to be used is a tyrosine kinase inhibitor as defined elsewhere herein. Preferably, said tyrosine kinase inhibitor is to be used in combination with the transduction enhancer as defined elsewhere herein.

Preferably, said subject being in need of receptor-targeted gene transfer suffers from (i) cancer, preferably leukemia, more preferably lymphoblastic leukemia, or (ii) an infectious disease, preferably viral infection, more preferably, HIV infection.

The present invention also contemplates a method for treating a subject in need thereof with receptor-targeted gene transfer, said method comprising:
(i) administering to said subject a therapeutically effective amount of a gene transfer vehicle comprising a targeting agent which specifically binds to CD3, CD4 or CD8 and a nucleic acid of interest to be transferred into primary T-cells into primary T-cells as host cells and administering to said subject an amount of tyrosine kinase inhibitor being capable of inhibiting the LCK tyrosine kinase in said primary T-cells, said amount being effective in enhancing the receptor-targeted gene transfer; or
(ii) administering to said subject a therapeutically effective amount of primary T-cells which have been obtained by the method of the present invention.

In the following, particular preferred embodiments of the present invention are described. The explanations and definitions of the terms apply mutatis mutandis.
Embodiment 1. A method for enhancing receptor-targeted gene transfer into a primary T-cell as a host cell said method comprising:
   (a) contacting a host cell comprised in a sample with a gene transfer vehicle comprising a targeting agent which specifically binds to CD3, CD4 or CD8 and a nucleic acid of interest to be transferred into the host cell in the presence of a tyrosine kinase inhibitor which is capable of inhibiting the LCK tyrosine kinase in said host cell; and
   (b) cultivating said host cell culture obtained in step (a) for a time and under conditions which allow for receptor-targeted gene transfer.
Embodiment 2. The method of embodiment 1, wherein said tyrosine kinase inhibitor is a Src/Abl tyrosine kinase inhibitor.
Embodiment 3. The method of embodiment 1 or 2, wherein said tyrosine kinase inhibitor is selected from the group consisting of: dasatinib, imatinib, and nilotinib.
Embodiment 4. The method of any one of embodiments 1 to 3, wherein said host cell is contacted during step (a) and/or step (b) with at least one further transduction enhancer.
Embodiment 5. The method of embodiment 4, wherein said further transduction enhancer is selected from the group consisting of: Vectofusin-1, BX 795, Cyclosporin A, CyclosporinH, 16,16-Dimethyl Prostaglandin E2, Prostaglandin E2, Rapamycin, Rosuvastin calcium, and Staurosporine.
Embodiment 5. The method of any one of embodiments 1 to 5, wherein said primary T-cell is a CD8 positive, CD4 positive or CD3 positive primary T-cell.
Embodiment 6. The method of any one of embodiments 1 to 5, wherein said receptor-targeted gene transfer into a primary T-cell as a host cell is enhanced at least 2-fold, at least 3 fold, at least 4 fold, up to 10 fold, preferably, at least 3 fold, compared to a control wherein the host cell was not contacted in the presence of said tyrosine kinase inhibitor.
Embodiment 7. A method for the preparation of a cellular therapeutic composition comprising the steps of the method of any one of embodiments 1 to 6 and the further step of formulating the host cell culture obtained in step (b) as a cellular therapeutic composition.
Embodiment 8. The method of embodiment 7, wherein said cellular therapeutic composition is for treating (i) cancer, preferably leukemia, more preferably lymphoblastic leukemia, or (ii) an infectious disease, preferably viral infection, more preferably, HIV infection.
Embodiment 9. A medicament comprising:
   (a) a gene transfer vehicle comprising a targeting agent which specifically binds to CD3, CD4 or CD8 and a nucleic acid of interest to be transferred into primary T-cells; and
   (b) tyrosine kinase inhibitor which is capable of inhibiting the LCK tyrosine kinase in said primary T-cells.
Embodiment 10. The medicament of embodiment 9, wherein said tyrosine kinase inhibitor is a tyrosine kinase inhibitor as defined in embodiment 2 or 3
Embodiment 11. The medicament of embodiment 9 or 10, wherein said medicament further comprises an transduction enhancer as defined in claim 5.
Embodiment 12. A tyrosine kinase inhibitor for use in enhancing receptor-targeted gene transfer into a primary T-cell in a subject being in need thereof, wherein said tyrosine kinase inhibitor is capable of inhibiting the LCK tyrosine kinase in a primary T-cell as a host cell and wherein a gene transfer vehicle comprising a targeting agent which specifically binds to CD3, CD4 or CD8 and a nucleic acid of interest to be transferred into the host cell.
Embodiment 13. A gene transfer vehicle comprising a targeting agent which specifically binds to CD3, CD4 or CD8 and a nucleic acid of interest to be transferred into the host cell for use in enhancing receptor-targeted gene transfer into a primary T-cell in a subject being in need thereof, wherein the gene transfer vehicle is used in combination with a tyrosine kinase inhibitor which is capable of inhibiting the LCK tyrosine kinase in a primary T-cell as a host cell.
Embodiment 14. The tyrosine kinase inhibitor for use of embodiment 12 or the gene transfer vehicle for use of claim 13, wherein said tyrosine kinase inhibitor is a tyrosine kinase inhibitor as defined in embodiment 2 or 3.
Embodiment 15. The tyrosine kinase for use of embodiment 12, the gene transfer vehicle for use of embodiment 13 or the tyrosine kinase inhibitor for use or gene transfer vehicle for use of embodiment 14, wherein said tyrosine kinase inhibitor is to be used in combination with the transduction enhancer as defined in embodiment 5.
Embodiment 16. The tyrosine kinase for use of embodiment 12, the gene transfer vehicle for use of embodiment 13 or the tyrosine kinase inhibitor for use or gene transfer vehicle for use of embodiment 14 or 15, wherein said subject being in need of receptor-targeted gene transfer suffers from (i) cancer, preferably leukemia, more preferably lymphoblastic leukemia, or (ii) an infectious disease, preferably viral infection, more preferably, HIV infection.
Embodiment 17. A method for treating a subject in need thereof with receptor-targeted gene transfer, said method comprising:
   (i) administering to said subject a therapeutically effective amount of a gene transfer vehicle comprising a targeting agent which specifically binds to CD3, CD4 or CD8 and a nucleic acid of interest to be transferred into primary T-cells into primary T-cells as host cells and administering to said subject an amount of tyrosine kinase inhibitor being capable of inhibiting the LCK tyrosine kinase in said primary T-cells, said amount being effective in enhancing the receptor-targeted gene transfer; or
   (ii) administering to said subject a therapeutically effective amount of primary T-cells which have been obtained by the method of any one of embodiments 1 to 8.

All references cited in this specification are herewith incorporated by reference with respect to their entire disclosure content and the disclosure content specifically mentioned in this specification.

### FIGURES

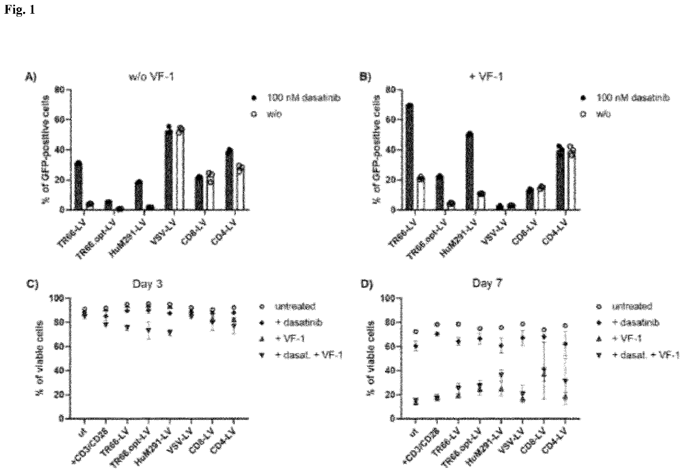

**Figure 1****:** Dasatinib enhances CD3-targeted gene transfer in activated PBMC. Freshly thawed primary human PBMC were activated for three days with αCD3 and αCD28 antibodies in presence of IL-2 prior to transduction with targeted LVs or VSV-LV, or without transduction remaining untransduced (ut). Cells were incubated in 100 nM dasatinib-containing media for 7 h during transduction. Additionally, VF-1 was added during transduction (B). Scatter bar diagrams summarize GFP-expression 3 days post transduction, comparing transduction in presence of dasatinib (filled dots) to transduction without (w/o) dasatinib (blank dots) (A-B). The cell viability was measured 3 (C) and 7 days (D) post transduction and is shown as superimposed symbols defining the different treatment conditions. Mean ±SD of three technical replicates.

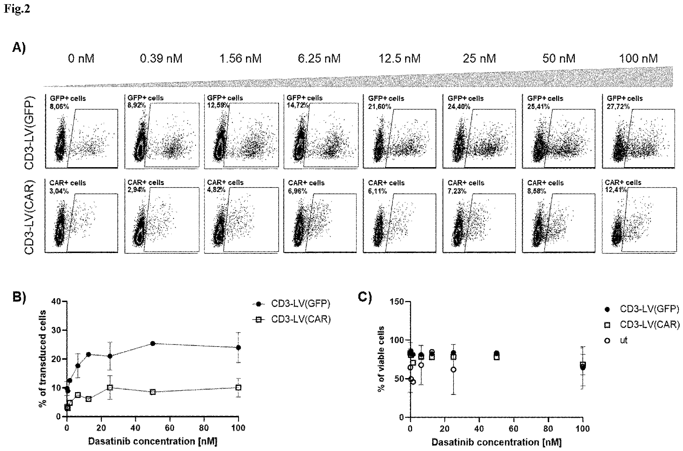

**Figure 2****:** Dasatinib enhances CD3-targeted gene transfer to activated PBMC in a dose-dependent manner. Freshly thawed primary human PBMC were activated for three days with αCD3 and αCD28 antibodies in presence of IL-7/15 prior to transduction with CD3-targeted LVs, or without transduction remaining untransduced (ut). Cells were incubated in 0.39-100 nM dasatinib-containing media for 7 h during transduction. Exemplary dot plots show dose-dependent transgene expression in T-cells, transduced with different dasatinib concentrations on day 3 post transduction (A). Superimposed symbols connected by line show dose-dependent transgene expression 3 days post transduction, with CD3-LV(GFP) (filled dots) and CD3-LV(CAR) (blank squares) (B). The cell viability was measured 3 days post transduction and is shown as superimposed symbols defining the different transduction conditions (C). N=1-2 with mean ±SD is shown for biological replicates.

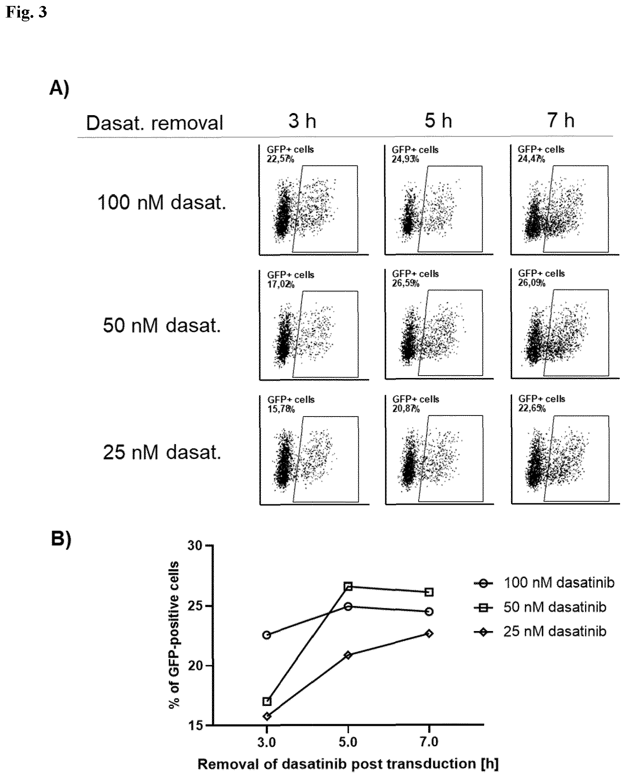

**Figure 3****:** Dasatinib enhances gene transfer in a time- and dose-dependent manner. Freshly thawed primary human PBMC were activated for three days with αCD3 and αCD28 antibodies in presence of IL-7/15 prior to transduction with CD3-targeted LV. Cells were incubated in 25 nM, 50 nM or 100 nM dasatinib-containing media for 3 h, 5 h or 7 h during transduction. Dot plots show dose- and time-dependent transgene expression in T-cells, transduced with different dasatinib concentrations and incubation times on day 3 post transduction (A). Superimposed symbols for different concentrations connected by line show GFP-expression 3 days post transduction, defining the different treatment conditions (B).

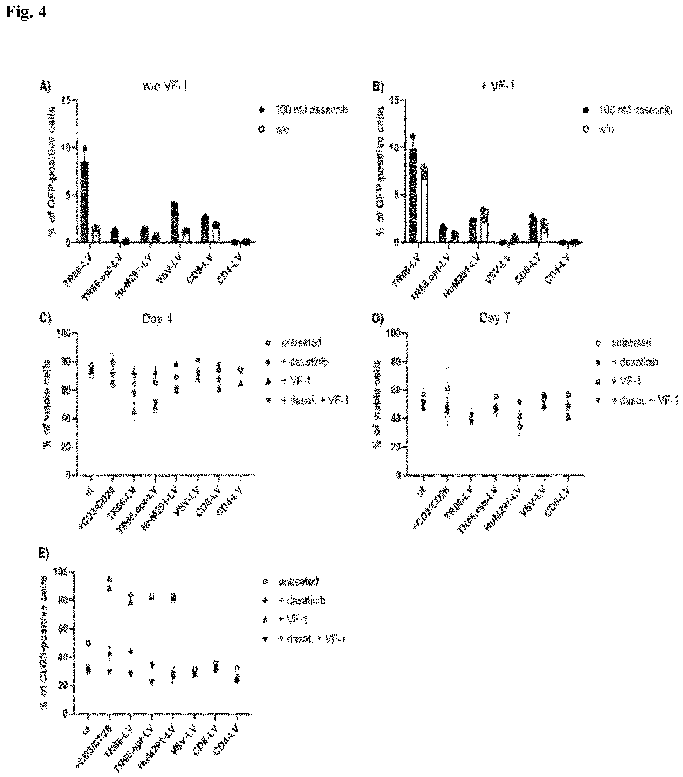

**Figure 4****:** Dasatinib enhances CD3-targeted transduction of minimally stimulated T-cells. Primary human PBMC were cultured in TCM supplemented only with IL-7/15 overnight and transduced the next day with targeted-LVs or VSV-LV, or remained untransduced (ut). Cells were incubated in 100 nM dasatinib-containing media for 7 h during transduction. Additionally, VF-1 was added during transduction (B). GFP-expression was determined 4 days later by flow cytometry and is shown as scatter bar diagram, comparing transduction in presence of dasatinib (filled dots) to transduction without (w/o) dasatinib (blank dots) (A-B). The cell viability was measured 4 (C) and 7 days (D) post transduction and is shown as superimposed symbols with a color code defining the different treatment conditions. On day 7, CD4-LV was excluded as the viability was too low (D). Expression of the activation marker CD25 is shown as superimposed symbols defining the different treatment conditions 4 days post transduction (E). Mean ±SD of three technical replicates.

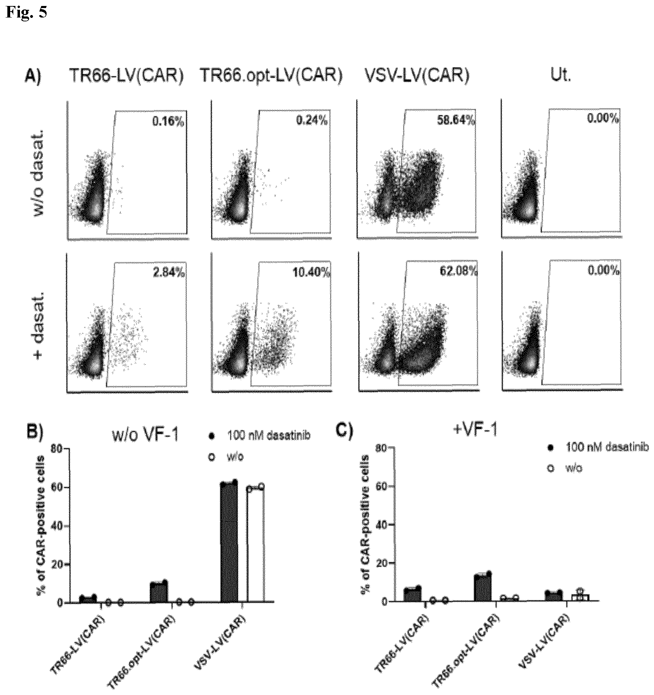

**Figure 5****:** Dasatinib enhances CD3-targeted CAR gene transfer to activated PBMC. Freshly thawed primary human PBMC were activated for three days with αCD3 and αCD28 antibodies in presence of IL-2 prior to transduction with TR66-LV, TR66.opt- or VSV-LV delivering a CD19-CAR, or without transduction remaining untransduced (ut). Cells were incubated in 100 nM dasatinib containing media for 7 h during transduction. Additionally, VF-1 was added during transduction (C). Exemplary dot plots show CAR-expression in CD3+ T-cells, transduced without VF-1 on day 3 post transduction (A). Percent of CAR-expression determined 3 days post transduction by flow cytometry is shown as scatter bar diagrams (B-C). Transduction in presence of dasatinib is shown as filled dots and transduction without (w/o) dasatinib is shown as blank dots. Mean ±SD of two technical replicates.

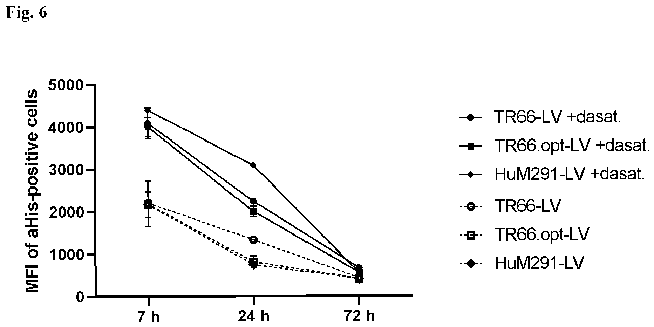

**Figure 6****:** Dasatinib enhances and prolongs binding of CD3-targeted LVs to activated PBMC. Freshly thawed primary human PBMC were activated for three days with αCD3 and αCD28 antibodies in presence of IL-2 prior to transduction with CD3-receptor-targeted LVs. Cells were incubated in 100 nM dasatinib-containing media for 7 h during transduction or without. Timelines show mean fluorescent intensity (MFI) of anti-His staining over 72h post treatment with dasatinib, allowing detection of LVs bound to PBMC. The filled symbols and lines show the binding of LVs to the cells treated with dasatinib (+dasat.) and the blank dots connected with the dashed lines show binding of LVs to cell not treated with dasatinib. Mean ±SD of three technical replicates.

### EXAMPLES

The Examples merely illustrate the invention and shall not be construed, whatsoever, as limiting the scope of protection.

### Example 1: Dasatinib enhances CD3-targeted transduction of fully activated PBMC in a time- and dose dependent manner.

To evaluate the effect of dasatinib on CD3-targeted transduction, PBMC activated for three days in presence of anti- (α) CD3 and αCD28 antibodies in TCM supplemented with IL-2 or IL-7/15 were incubated with three CD3-targeted LVs, namely TR66-LV, TR66.opt-LV and HuM291-LV, as well as CD4-LV, CD8-LV or VSV-LV as control. Cells received between 0.39 and 100 nM dasatinib-containing media one hour prior to transduction, which was removed from the cells 3, 5 or 7 h post-transduction. In particular, human peripheral blood mononuclear cells (PBMC) were isolated from blood of health and anonymous donors who had given their consent. Cells were either thawed and activated with anti-human CD3 (aCD3; clone OKT3) and anti-human CD28 (aCD28; 15E8, both Miltenyi Biotec) for three days, or, used freshly after isolation and overnight incubation in T-cell media (TCM; complete RPMI containing 0.5% penicillin/streptomycin and 25 mM HEPES (Sigma-Aldrich)) supplemented with IL-2 (50 U/ml) or IL-7 (25 U/ml) and IL-15 (U/ml) (all Miltenyi Biotec). For enhanced T-cell transduction of PBMC in presence of dasatinib (Selleck Chemicals, reconstituted in DMSO), 8x10e4 cells/well of fully activated or IL-7/15 stimulated PBMC were seeded in a 96-well plate. The cells were seeded in 100 µL TCM supplemented with IL-2 (50 U/ml) or IL-7 (25 U/ml) and IL-15 (5 U/ml) and one hour prior to transduction 0.39-100 nM dasatinib was added to the culturing media. The cells were transduced with 5 µL of the appropriate lentiviral vector · stock (particle number/ MOI), following spinfection via centrifugation for 90 min at 850xg and 32°C.

Transduction was additionally promoted by the transduction enhancer VF-1. Culture medium without dasatinib and Vectofusin-1 was used for control transductions. Transgene expression in T-cells was evaluated 3 and 7 days post transduction by flow cytometry. In particular, for further enhancement of transduction in some experiments, Vectofusin-1 (VF-1) (Miltenyi Biotec) was incubated with the vectors for 5-10 min prior to adding the transduction mix to the cells and performing spinfection. After spinfection, 100 µL of fresh media, either containing the different dasatinib concentrations or without dasatinib, was added and the cells were cultured at 37°C. The medium was changed to fresh TCM with IL-2 or IL-7/15 without dasatinib 3 h, 5 h or 7 h after start of incubation with dasatinib. The cells were further cultured at 37°C and analyzed for transgene expression 3-4 and 7 days post transduction by flow cytometry.

Transduction by all three CD3-targeted LVs (TR66-, TR66.opt- and HuM291-LV) was increased by 5- to 10-fold in presence of dasatinib. Transduction by CD8-LV and VSV-LV was not affected, and CD4-LV transduction showed a slight increase in gene transfer (Figure 1 A-B). In untreated conditions, the CD3-targeted TR66-LV reached 4% GFP-expression which was increased to 30% GFP-expression in presence of dasatinib (Figure 1 A). When VF-1 was added during transduction in presence of dasatinib, an even higher transduction of nearly 70% for TR66-LV was achieved and an overall 3- to 5-fold increase in transduction could be observed for the CD3-targeted LVs (Figure 1 B). Gene transfer remained stable upon 7 days post-transduction in all conditions. Also, viability was not compromised in presence of dasatinib compared to untreated cells, but treatment with VF-1 and VF-1 in combination of dasatinib led to an extreme loss of viability on day 7 post transduction (Figure 1C-D).

Transduction enhancement positively correlated with increased dasatinib concentration. The lowest concentration of 0.39 nM dasatinib was not sufficient to enhance transduction with CD3-targeted LVs, while starting at 1.56 nM dasatinib transduction showed a 1.6-fold increase compared to untreated cells (Figure 2). Concentrations ≥50 nM reached a 3-fold increase in transduction which, in this setup, seemed to be the maximum one could reach. At dasatinib concentrations above 50 nM viability of the cells dropped slightly (Figure 2C). Additionally, incubation time of the cells with dasatinib affected transduction enhancement. Longer treatment of 5-7 h increased transduction enhancement, while removal of dasatinib-containing media after 3 h resulted in 10-35% less transduction compared to longer incubation time (Figure 3). These results prove that incubating activated T-cells for 5 h with media containing 50 nM dasatinib is the optimal condition for CD3-targeted LV transduction, enabling enhanced gene transfer compared to transduction in absence of dasatinib.

### Example 2: Dasatinib enhances CD3-targeted transduction of minimally stimulated PBMC

In a next step, freshly isolated PBMC exposed solely to IL-7/15 overnight, were incubated with 100 nM dasatinib one hour prior to incubation with vector particles in presence or absence of VF-1. Similar to fully activated PBMC, dasatinib enhanced transduction by CD3-targeted LVs 3- to 8-fold (Figure 4A). The addition of VF-1 to dasatinib-treated cells during transduction increased TR66-LV mediated gene transfer to 10%, which is a smaller increase compared to activated PBMC (Figure 4B). Importantly, dasatinib did not negatively affecT-cell viability of non-activated cells, and compared to activated PBMC, cells treated with VF-1 exhibited sufficient viability at day 7 (Figure 4 C-D). By tracking the expression of the activation marker CD25, 4 days post transduction, a difference in activation status could be observed between CD3-LV-transduced cells treated with dasatinib compared to CD3-LV-transduced cells without dasatinib (Figure 4 E). Similar to cells incubated with activating antibodies, CD3-LV-treated cells showed an upregulation of CD25 expression to above 80% compared to the background expression of around 30-50%. In presence of dasatinib, however, incubation with activating antibodies or CD3-LVs did not cause an increase in CD25-expression. These results emphasize the interference in T-cell activation known to be induced by dasatinib through inhibition of the tyrosine kinase LCK 1,2.

### Example 3: Dasatinib increases chimeric antigen receptor (CAR) gene transfer by CD3-targeted LVs

To verify that the observed increase in transduction caused by dasatinib is applicable for CAR gene transfer, activated PBMC were incubated with TCM +IL-2 supplemented with 100 nM dasatinib and transduced with two CD3-targeted LVs, TR66-LV and TR66.opt-LV, or VSV-LV delivering CD19-CAR containing a myc tag for detection. Flow cytometry 3 days post transduction revealed low CAR expression on CD3-LV transduced cells of below 0.3%, which was increased to up to 10% CAR-expression when dasatinib was present during transduction (Figure 5). Also in presence of VF-1, dasatinib increased transduction from 1.6% to 13% (Figure 5C).

### Example 4: Dasatinib enhances binding of CD3-targeted LVs to activated PBMC

To understand the mechanism behind the transduction enhancing characteristic of dasatinib, fully activated PBMC were incubated with CD3-targeted LVs, as described above, and analyzed via flow cytometry 7, 24 and 72 h after start of incubation with dasatinib and transduction. Cells were stained with anti-His-PE, allowing the detection of LVs bound to the cells.

The amount of CD3-LVs bound to the cells was substantially increased when cells had been treated with dasatinib. This was especially pronounced at the early time point (7 h after start of dasatinib treatment) and also detectable after 24 h when vector binding in absence of dasatinib had returned to background level (Figure 6). This finding suggests, that dasatinib enhances the amount of targeted receptor, in this case CD3, on the cell surface and prolongs its presence, thus allowing more CD3-targeted LVs to bind to targeT-cells, which results in better transduction.

### CITED LITERATURE

Agarwal, Shiwani; Hanauer, Julia D. S.; Frank, Annika M.; Riechert, Vanessa; Thalheimer, Frederic B.; Buchholz, Christian J. (2020): In Vivo Generation of CAR T-cells Selectively in Human CD4+ Lymphocytes. In: Mol. Ther. 28, 1783-1794. DOI: 10.1016/j.ymthe.2020.05.005.
Frank, Annika M.; Braun, Angela H.; Scheib, Lea; Agarwal, Shiwani; Schneider, Irene C.; Fusil, Floriane et al. (2020): Combining T-cell specific activation and in vivo gene delivery through CD3-targeted lentiviral vectors. In: Blood Adv. DOI: 10.1182/bloodadvances.2020002229.
Jamali, Arezoo; Kapitza, Laura; Schaser, Thomas; Johnston, Ian C. D.; Buchholz, Christian J.; Hartmann, Jessica (2019): Highly Efficient and Selective CAR-Gene Transfer Using CD4- and CD8-Targeted Lentiviral Vectors. In: Mol. Ther. Methods. Clin. Dev. 13, S. 371-379. DOI: 10.1016/j.omtm.2019.03.003.
Lissina, Anna; Ladell, Kristin; Skowera, Ania; Clement, Matthew; Edwards, Emily; Seggewiss, Ruth et al. (2009): Protein kinase inhibitors substantially improve the physical detection of T-cells with peptide-MHC tetramers. In: J. Immunol. Methods 340 (1), S. 11-24. DOI: 10.1016/j.jim.2008.09.014.
Mestermann, Katrin; Giavridis, Theodoros; Weber, Justus; Rydzek, Julian; Frenz, Silke; Nerreter, Thomas et al. (2019): The tyrosine kinase inhibitor dasatinib acts as a pharmacologic on/off switch for CAR T-cells. In: Sci Transl Med 11 (499). DOI: 10.1126/scitranslmed.aau5907.
Pfeiffer, Anett; Thalheimer, Frederic B.; Hartmann, Sylvia; Frank, Annika M.; Bender, Ruben R.; Danisch, Simon et al. (2018): In vivo generation of human CD19-CAR T-cells results in B-cell depletion and signs of cytokine release syndrome. In: EMBO Mol Med 10 (10), e9158. DOI: 10.15252/emmm.201809158.
Schade, Andrew E.; Schieven, Gary L.; Townsend, Robert; Jankowska, Anna M.; Susulic, Vojkan; Zhang, Rosemary et al. (2008): Dasatinib, a small-molecule protein tyrosine kinase inhibitor, inhibits T-cell activation and proliferation. In: Blood 111 (3), S. 1366-1377. DOI: 10.1182/blood-2007-04-084814.
Shangming Zhang, Guiandre Joseph, Karen Pollok, Lionel Berthoux, Lakshmi Sastry, Jeremy Luban, and Kenneth Cornetta (2006): G2 Cell Cycle Arrest and Cyclophilin A in Lentiviral Gene Transfer. Molecular Ther. (2006) 14,(4): 546.
WO2019/110815
WO2020/092650,
WO2019/213184
WO2018/183842
US2020/181573
WO2005077945
WO2006121742
Binz HK, Stumpp MT, Forrer P, Amstutz P, Plückthun A (September 2003). "Designing repeat proteins: well-expressed, soluble and stable proteins from combinatorial libraries of consensus ankyrin repeat proteins". Journal of Molecular Biology. 332 (2): 489-503.

## Claims

**1.** A method for enhancing receptor-targeted gene transfer into a primary T-cell as a host cell said method comprising:
(a) contacting a host cell comprised in a sample with a gene transfer vehicle comprising a targeting agent which specifically binds to CD3, CD4 or CD8 and a nucleic acid of interest to be transferred into the host cell in the presence of a tyrosine kinase inhibitor which is capable of inhibiting the LCK tyrosine kinase in said host cell; and
(b) cultivating said host cell culture obtained in step (a) for a time and under conditions which allow for receptor-targeted gene transfer.

**2.** The method of claim 1, wherein said tyrosine kinase inhibitor is a Src/Abl tyrosine kinase inhibitor.

**3.** The method of claim 1 or 2, wherein said tyrosine kinase inhibitor is selected from the group consisting of: dasatinib, imatinib, and nilotinib.

**4.** The method of any one of claims 1 to 3, wherein said host cell is contacted during step (a) and/or step (b) with at least one further transduction enhancer.

**5.** The method of claim 4, wherein said further transduction enhancer is selected from the group consisting of: Vectofusin-1, BX 795, Cyclosporin A, CyclosporinH, 16,16-Dimethyl Prostaglandin E2, Prostaglandin E2, Rapamycin, Rosuvastin calcium, and Staurosporine.

**5.** The method of any one of claims 1 to 5, wherein said primary T-cell is a CD8 positive, CD4 positive or CD3 positive primary T-cell.

**6.** The method of any one of claims 1 to 5, wherein said receptor-targeted gene transfer into a primary T-cell as a host cell is enhanced at least 2-fold, at least 3 fold, at least 4 fold, up to 10 fold, preferably, at least 3 fold, compared to a control wherein the host cell was not contacted in the presence of said tyrosine kinase inhibitor.

**7.** A method for the preparation of a cellular therapeutic composition comprising the steps of the method of any one of claims 1 to 6 and the further step of formulating the host cell culture obtained in step (b) as a cellular therapeutic composition.

**8.** The method of claim 7, wherein said cellular therapeutic composition is for treating (i) cancer, preferably leukemia, more preferably lymphoblastic leukemia, or (ii) an infectious disease, preferably viral infection, more preferably, HIV infection.

**9.** A medicament comprising:
(a) a gene transfer vehicle comprising a targeting agent which specifically binds to CD3, CD4 or CD8 and a nucleic acid of interest to be transferred into primary T-cells; and
(b) tyrosine kinase inhibitor which is capable of inhibiting the LCK tyrosine kinase in said primary T-cells.

**10.** The medicament of claim 9, wherein said tyrosine kinase inhibitor is a tyrosine kinase inhibitor as defined in claim 2 or 3

**11.** The medicament of claim 9 or 10, wherein said medicament further comprises an transduction enhancer as defined in claim 5.

**12.** A tyrosine kinase inhibitor for use in enhancing receptor-targeted gene transfer into a primary T-cell in a subject being in need thereof, wherein said tyrosine kinase inhibitor is capable of inhibiting the LCK tyrosine kinase in a primary T-cell as a host cell and wherein a gene transfer vehicle comprising a targeting agent which specifically binds to CD3, CD4 or CD8 and a nucleic acid of interest to be transferred into the host cell.

**13.** A gene transfer vehicle comprising a targeting agent which specifically binds to CD3, CD4 or CD8 and a nucleic acid of interest to be transferred into the host cell for use in enhancing receptor-targeted gene transfer into a primary T-cell in a subject being in need thereof, wherein the gene transfer vehicle is used in combination with a tyrosine kinase inhibitor which is capable of inhibiting the LCK tyrosine kinase in a primary T-cell as a host cell.

**14.** The tyrosine kinase inhibitor for use of claim 12 or the gene transfer vehicle for use of claim 13, wherein said tyrosine kinase inhibitor is a tyrosine kinase inhibitor as defined in claim 2 or 3.

**15.** The tyrosine kinase for use of claim 12, the gene transfer vehicle for use of claim 13 or the tyrosine kinase inhibitor for use or gene transfer vehicle for use of claim 14, wherein said tyrosine kinase inhibitor is to be used in combination with the transduction enhancer as defined in claim 5.

**16.** The tyrosine kinase for use of claim 12, the gene transfer vehicle for use of claim 13 or the tyrosine kinase inhibitor for use or gene transfer vehicle for use of claim 14 or 15, wherein said subject being in need of receptor-targeted gene transfer suffers from (i) cancer, preferably leukemia, more preferably lymphoblastic leukemia, or (ii) an infectious disease, preferably viral infection, more preferably, HIV infection.
